# EUROPEAN PATENT SPECIFICATION

(11) **EP 3 888 613 B1**
(45) Date of publication and mention of the grant of the patent: **10.04.2024**
(21) Application number: 21176832.0
(22) Date of filing: 18.02.2020
(51) Int. Cl.: A61H 31/00, A61N 1/39

(54) **DEFIBRILLATOR PROVIDING CARDIO PULMONARY RESUSCITATION FEEDBACK**
DEFIBRILLATOR MIT BEREITSTELLUNG VON FEEDBACK HINSICHTLICH DER HERZ-LUNGEN-REANIMATION
DÉFIBRILLATEUR FOURNISSANT UNE RÉTROACTION DE RÉANIMATION CARDIO-PULMONAIRE

(30) Priority: 25.02.2019 GB 201902526
(43) Date of publication of application: 06.10.2021
(62) Divisional of application: 20157896.0
(73) Proprietor: Stryker European Operations Limited, Carrigtwohill, Co. Cork T45 HX08 (IE)
(72) Inventor: McAlister, Olibhear, Belfast, BT3 9ED (GB); Donaghy, Dymphna, Belfast, BT3 9ED (GB); Anderson, Johnny, Belfast, BT3 9ED (GB); Harvey, Adam, Belfast, BT3 9ED (GB); Torney, Hannah, Belfast, BT3 9ED (GB)
(74) Representative: HGF

(56) References cited:
- EP-A1- 2 653 146
- WO-A1-2012/162048
- CN-A- 107 625 627
- US-A1- 2006 173 501
- US-A1- 2012 010 543
- US-A1- 2014 277 224

## Description

The invention relates to a defibrillator providing cardio pulmonary resuscitation (CPR) feedback to a person during the performance of CPR on a subject.

Various circumstances may arise when a defibrillator instructs a person to carry out CPR on a subject. CPR involves the compression of the subject's chest to cause the heart to pump blood around their circulatory system, primarily to provide oxygenated blood to the subject's heart and brain. If the compression of the chest is too shallow, then the heart will not pump sufficient oxygenated blood and the heart and brain tissue will start to die. For effective CPR, guidelines recommend a specific target depth of compression of the subject's chest. Typically, however, most people do not achieve this target depth. Defibrillators which provide feedback based on comparison of CPR performance of the person with a guideline-set target depth can discourage the person if they do not reach the target. Feedback based on comparison of a person's CPR performance during a number of CPR periods can improve CPR performance.

US2012/010543 describes systems and techniques that may be used to provide information about patient status and/or CPR administration during administration of CPR to a patient. The systems and techniques aim to identify the most important data and to display the information in an efficient and effective manner to a rescuer. The data displayed to the rescuer can include information about the quality of the CPR administered by the rescuer, including, for example, CPR chest compression depth. The data displayed to the rescuer can also include information about the patient status. The data about the patient and CPR is presented graphically and/or textually in a manner that improves the ability of a rescuer to quickly understand the state of a patient and to make clinical decisions that will benefit the patient.

US2014/277224 discloses a system for managing care of a person receiving emergency cardiac assistance that includes one or more capacitors for delivering a defibrillating shock to a patient; one or more electronic ports for receiving signals from sensors for obtaining indications of an electrocardiogram (ECG) for the patient; a patient treatment module executable on one or more computer processors to provide a determination of a likelihood of success from delivering a future defibrillating shock to the person with the one or more capacitors, using (a) information about a prior defibrillating shock, and (b) a value that is a function of current ECG signals from the patient.

US2006/173501 discloses a method for assessing CPR performed during at least one resuscitation event which includes obtaining a data set during each of the at least one resuscitation events, each data set including data characterizing the performance of chest compressions or ventilations, or both, on the patient during the at least one resuscitation event; processing the data set to determine a value of at least one parameter of the performance; and displaying a graphical representation of the at least one parameter. A graphical representation of a desired value or range of values of the at least one parameter may be displayed in a position for visual comparison with the determined value. A method for presenting information on a sequence of events during a resuscitation event includes: obtaining data on the sequence and timing of chest compression and lung ventilations from a defibrillator; and displaying symbols for chest compressions and lung ventilations in a linear sequence corresponding to the order in which the compressions and ventilations occurred, the linear distance between successive symbols being proportional to the temporal distance between the corresponding actions.

EP2653146 discloses an automated external defibrillator (AED) and methods for a corrective CPR prompting system. The AED includes a sensor that obtains compression measurement data of CPR chest compressions and a control system including a microprocessor programmed to run a non-parametric, Information-Theoretic analysis of the compression measurement data. The analysis includes ranking provided compression measurement data, determining a prompt time TN for review, locating the compression measurement data at TN in an initial expected histogram of depth and rate aspects of the compression data measurements with upper and lower limits, that divides the intervals of the histogram intoa plurality of sections, weighting the compression measurement data based on a plurality of factors, deriving information content of the compression measurement data by mapping a probability density function into an information content function, and determining if a particular corrective prompt is necessary. The AED also includesa prompting device that provides corrective CPR.

WO2012/162048 discloses a medical system comprises a manual patient ventilation unit defining an airflow path. The unit is arranged so that when the unit is applied to a patient, the airflow path is in fluid communication with the patient's airway. The patient ventilation unit comprises a ventilation bag configured to enable manual ventilation of the patient by a rescuer; an airflow sensor in the airflow path positioned to sense the presence of ventilation airflow and measure a gas flow rate in the airflow path, a pressure sensor in the airflow path positioned to sense gas pressure in the airflow path, and a processor arranged to receive data generated by the airflow sensor and the pressure sensor and determine one or more ventilation quality parameters based at least in part on a gas flow volume calculated based on the sensed gas flow rate and gas pressures. A feedback unit provides feedback to a rescuer based on the one or more ventilation quality parameters.

CN107625627 discloses a CPR auxiliary system which comprises an ECG sensor, a PPG sensor, a pressure sensor, and a displacement sensor. An ECG signal, a blood oxygen impulse wave signal, a compression force signal and a compression depth signal are measured for a patient receiving CPR. Four sensors are connected with an intelligent controller through a serial port, and the intelligent controller can process the signals during an compression defibrillation rhythm identifying module, an external chest compression depth optimizing module and an autonomous circular identifying module, so as to obtain autonomous recovery prompt information, individualized optimal compression depth prompt information, and uninterrupted external chest compression defibrillation rhythm prompt information for the patient. This information can be displayed through a man-machine interactive interface or in a voice form through a loudspeaker. The CPR auxiliary system can provide optimal suggestions for treatment according to the vital signs of the patient, so that the CPR quality is effectively improved.

In each of the above documents, CPR performance targets are set with reference to guidelines or a physician, or are set at a desired or acceptable value or range. The target may be changed according to measured patient parameters indicating the effectiveness of the CPR on the patient.

It is an object of the present invention to provide a defibrillator which assesses CPR carried out by a person on a subject and provides CPR feedback to the person based on the person's CPR performance.

According to the invention there is provided a defibrillator which assesses cardio pulmonary resuscitation (CPR) carried out by a person on a subject and provides CPR feedback to the person, comprising:
a biosignal measurement system configured to measure biosignals of the subject, determine when CPR is required and produce a CPR start signal and determine and when CPR is to be ceased and produce a CPR stop signal,
a CPR measurement system configured to measure compression signals during CPR chest compressions by the person,
a CPR assessment system connected to the biosignal measurement system to receive the CPR start signal and the CPR stop signal, connected to the CPR measurement system to receive compression signals and configured to perform the steps:
   (i) receive the CPR start signal and produce a first feedback signal,
   (ii) receive compression signals measured during a first plurality of chest compressions by the person,
   (iii) use the compression signals to establish a first CPR performance measurement of the person,
   (iv) set a CPR performance baseline of the person equal to the first CPR performance measurement of the person,
   (v) receive compression signals measured during a further plurality of chest compressions by the person,
   (vi) use the compression signals to establish a further CPR performance measurement of the person,
   (vii) compare the further CPR performance measurement of the person with the CPR performance baseline of the person,
   (viii) when the further CPR performance measurement of the person is greater than the CPR performance baseline of the person, produce a second feedback signal and set the CPR performance baseline of the person equal to the further CPR performance measurement of the person and go to step (x),
   (ix) when the further CPR performance measurement of the person is less than the CPR performance baseline of the person, produce a third feedback signal and go to step (x),
   (x) when the CPR stop signal is not received, return to step (v),
   (xi) when the CPR stop signal is received, produce a fourth feedback signal,
   and
a feedback unit connected to the CPR assessment system and configured to receive the feedback signals and issue CPR feedback to the person.

The defibrillator measures the CPR performance of the person during a plurality of CPR chest compressions and uses self-comparison of the person's CPR performance to provide appropriate CPR feedback.

Steps (viii) to (x) may further comprise:
(viii) when the further CPR performance measurement is greater than the CPR performance baseline, produce the second feedback signal and set the CPR performance baseline equal to the further CPR performance measurement and go to step (x)(a),
(ix) when the further CPR performance measurement is less than the CPR performance baseline, produce the third feedback signal and go to step (x)(b),
(x)(a) when the CPR stop signal is not received, return to step (v),
(x)(b) when the CPR stop signal is not received, wait for a plurality of chest compressions, return to step (v).

Steps (viii) to (x) may further comprise:
(viii) when the further CPR performance measurement is greater than the CPR performance baseline, produce the second feedback signal and set the CPR performance baseline equal to the further CPR performance measurement and go to step (x)(a),
(ix) when the further CPR performance measurement is less than the CPR performance baseline, produce the third feedback signal and go to step (x)(b),
(x)(a) when the CPR stop signal is not received, wait for a plurality of chest compressions, return to step (v),
(x)(b) when the CPR stop signal is not received, return to step (v).

Steps (viii) to (x) may further comprise:
(viii) when the further CPR performance measurement is greater than the CPR performance baseline, produce the second feedback signal and set the CPR performance baseline equal to the further CPR performance measurement and go to step (x)(a),
(ix) when the further CPR performance measurement is less than the CPR performance baseline, produce the third feedback signal and go to step (x)(b),
(x)(a) when the CPR stop signal is not received, wait for a plurality of chest compressions, return to step (v),
(x)(b) when the CPR stop signal is not received, wait for a plurality of chest compressions, return to step (v).

Steps (ii) to (iv) may further comprise:
(ii) receive first compression signals measured during a first plurality of chest compressions by the person and at least second compression signals measured during a second plurality of chest compressions by the person,
(iii) use the first compression signals to establish a first CPR performance measurement and use the at least second compression signals to establish an at least second CPR performance measurement,
(iv) set a CPR performance baseline equal to the greater of the first CPR performance measurement and the at least second CPR performance measurement.

Steps (v) to (x) may further comprise:
(v)(a) set a CPR counter equal to x,
(v)(b) receive compression signals measured during a further plurality of chest compressions by the person,
(vi) use the compression signals to establish a further CPR performance measurement,
(vii) compare the further CPR performance measurement with the CPR performance baseline,
(viii) when the further CPR performance measurement is greater than the CPR performance baseline, produce the second feedback signal, set the CPR performance baseline equal to the further CPR performance measurement and go to (x)(a),
(ix)(a) when the further CPR performance measurement is less than the CPR performance baseline, when the CPR counter is not equal to zero, produce the third feedback signal, decrease the CPR counter by 1 and go to (x)(b),
(ix)(b) when the further CPR performance measurement is less than the CPR performance baseline, when the CPR counter is equal to zero, produce the second feedback signal, decrease the CPR performance baseline and go to (x)(a),
(x)(a) when the CPR stop signal is not received, return to step (v)(a)
(x)(b) when the CPR stop signal is not received, return to step (v)(b).

Decreasing the CPR performance baseline may comprise setting the CPR performance baseline equal to the further CPR performance measurement. Decreasing the CPR performance baseline may comprise setting the CPR performance baseline equal to a greater of further CPR performance measurements.

Step (x)(a) may comprise when the CPR stop signal is not received, wait for a plurality of compressions and return to step (v)(a). Step (x)(b) may comprise when the CPR stop signal is not received, wait for a plurality of compressions and return to step (v)(b).

Step (ii) may further comprise:
(ii)(a) set a CPR counter equal to x,
(ii)(b) receive compression signals measured during a first plurality of chest compressions by the person,
(ii)(c) receive a compression rate measured during the first plurality of chest compressions,
(ii)(d) compare the measured compression rate with a minimum required compression rate,
(ii)(e) when the measured compression rate is greater than the minimum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii).

Step (ii)(f) may comprise when the measured compression rate is less than the minimum required compression rate, when the CPR counter is not equal to zero, decrease the CPR counter by 1, produce a fifth feedback signal, wait for a plurality of compressions and go to step (ii)(b).

Step (ii) may further comprise:
(ii)(a) set a CPR counter equal to x,
(ii)(b) receive compression signals measured during a first plurality of chest compressions by the person,
(ii)(c) receive a compression rate measured during the first plurality of chest compressions,
(ii)(d) compare the measured compression rate with a maximum required compression rate,
(ii)(e) when the measured compression rate is less than the maximum required compression rate, go to step (iii),
(ii)(f) when the measured compression rate is greater than the maximum required compression rate, when the CPR counter is not equal to zero, decrease the CPR counter by 1, produce a sixth feedback signal and go to step (ii)(b),
(ii)(g) when the measured compression rate is greater than the maximum required compression rate, when the CPR counter is equal to zero, go to step (iii).

Step (ii)(f) may comprise when the measured compression rate is greater than the maximum required compression rate, when the CPR counter is not equal to zero, decrease the CPR counter by 1, produce a sixth feedback signal, wait for a plurality of compressions and go to step (ii)(b).

Step (ii) may further comprise:
(ii)(a) set a CPR counter equal to x,
(ii)(b) receive compression signals measured during a first plurality of chest compressions by the person,
(ii)(c) receive a compression rate measured during the first plurality of chest compressions,
(ii)(d) compare the measured compression rate with a minimum required compression rate and a maximum required compression rate,
(ii)(e) when the measured compression rate is greater than the minimum required compression rate and when the measured compression rate is less than the maximum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii), (ii)(h) when the measured compression rate is greater than the maximum required compression rate, when the CPR counter is not equal to zero, decrease the CPR counter by 1, produce a sixth feedback signal and go to step (ii)(b),
(ii)(i) when the measured compression rate is greater than the maximum required compression rate, when the CPR counter is equal to zero, go to step (iii).

Step (ii)(f) may comprise when the measured compression rate is less than the minimum required compression rate, when the CPR counter is not equal to zero, decrease the CPR counter by 1, produce a fifth feedback signal, wait for a plurality of compressions and go to step (ii)(b).

Step (ii)(h) may comprise when the measured compression rate is greater than the maximum required compression rate, when the CPR counter is not equal to zero, decrease the CPR counter by 1, produce a sixth feedback signal, wait for a plurality of compressions and go to step (ii)(b).

Step (v) may further comprise:
(v)(a) set a CPR counter equal to x,
(v)(b) receive compression signals measured during a further plurality of chest compressions by the person,
(v)(c) receive a compression rate measured during the further plurality of chest compressions,
(v)(d) compare the measured compression rate with a minimum required compression rate,
(v)(e) when the measured compression rate is greater than the minimum required compression rate, go to step (vi),
(v)(f) when the measured compression rate is less than the minimum required compression rate, when the CPR counter is not equal to zero, decrease the CPR counter by 1, produce a fifth feedback signal and go to step (v)(b),
(v)(g) when the measured compression rate is less than the minimum required compression rate, when the CPR counter is equal to zero, go to step (vi).

Step (v)(f) may comprise when the measured compression rate is less than the minimum required compression rate, when the CPR counter is not equal to zero, decrease the CPR counter by 1, produce a fifth feedback signal, wait for a plurality of compressions and go to step (v)(b).

Step (v) may further comprise:
(v)(a) set a CPR counter equal to x,
(v)(b) receive compression signals measured during a further plurality of chest compressions by the person,
(v)(c) receive a compression rate measured during the further plurality of chest compressions,
(v)(d) compare the measured compression rate with a maximum required compression rate,
(v)(e) when the measured compression rate is less than the maximum required compression rate, go to step (vi),
(v)(f) when the measured compression rate is greater than the maximum required compression rate, when the CPR counter is not equal to zero, decrease the CPR counter by 1, produce a sixth feedback signal and go to step (v)(b),
(v)(g) when the measured compression rate is greater than the maximum required compression rate, when the CPR counter is equal to zero, go to step (vi).

Step (v)(f) may comprise when the measured compression rate is greater than the maximum required compression rate, when the CPR counter is not equal to zero, decrease the CPR counter by 1, produce a sixth feedback signal, wait for a plurality of compressions and go to step (v)(b).

Step (v) may further comprise:
(v)(a) set a CPR counter equal to x,
(v)(b) receive compression signals measured during a further plurality of chest compressions by the person,
(v)(c) receive a compression rate measured during the further plurality of chest compressions,
(v)(d) compare the measured compression rate with a minimum required compression rate and a maximum required compression rate,
(v)(e) when the measured compression rate is greater than the minimum required compression rate and when the measured compression rate is less than the maximum required compression rate, go to step (vi),
(v)(f) when the measured compression rate is less than the minimum required compression rate, when the CPR counter is not equal to zero, decrease the CPR counter by 1, produce a fifth feedback signal and go to step (v)(b),
(v)(g) when the measured compression rate is less than the minimum required compression rate, when the CPR counter is equal to zero, go to step (vi),
(v)(h) when the measured compression rate is greater than the maximum required compression rate, when the CPR counter is not equal to zero, decrease the CPR counter by 1, produce a sixth feedback signal and go to step (v)(b),
(v)(i) when the measured compression rate is greater than the maximum required compression rate, when the CPR counter is equal to zero, go to step (vi).

Step (v)(f) may comprise when the measured compression rate is less than the minimum required compression rate, when the CPR counter is not equal to zero, decrease the CPR counter by 1, produce a fifth feedback signal, wait for a plurality of compressions and go to step (v)(b).

Step (v)(h) may comprise when the measured compression rate is greater than the maximum required compression rate, when the CPR counter is not equal to zero, decrease the CPR counter by 1, produce a sixth feedback signal, wait for a plurality of compressions and go to step (v)(b).

The CPR assessment system may be configured to perform a step (iii)(a) after step (iii) comprising compare the first CPR performance measurement of the person with a minimum CPR performance measurement, when the first CPR performance measurement is greater than the minimum CPR performance measurement go to step (iv), when the first CPR performance measurement is less than the minimum CPR performance measurement produce the third prompt signal and return to step (ii).

Step (iii)(a) may comprise when the first CPR performance measurement is less than the minimum CPR performance measurement, produce the third prompt signal, wait for a plurality of compressions and return to step (ii).

The CPR assessment system may be configured to perform a step (iii)(b) after step (iii)(a) comprising compare the first CPR performance measurement of the person with a maximum CPR performance measurement, when the first CPR performance measurement is less than the maximum CPR performance measurement go to step (iv), when the first CPR performance measurement is greater than the maximum CPR performance measurement produce a seventh prompt signal and return to step (ii).

Step (iii)(b) may comprise when the first CPR performance measurement is greater than the maximum CPR performance measurement, produce a seventh prompt signal, wait for a plurality of compressions and return to step (ii).

The CPR assessment system may be configured to perform a step (vi)(a) after step (vi) comprising compare the further CPR performance measurement of the person with a minimum CPR performance measurement, when the further CPR performance measurement is greater than the minimum CPR performance measurement go to step (vii), when the further CPR performance measurement is less than the minimum CPR performance measurement produce the third prompt signal and return to step (v).

Step (vi)(a) may comprise when the further CPR performance measurement is less than the minimum CPR performance measurement, produce the third prompt signal, wait for a plurality of compressions and return to step (v).

The CPR assessment system may be configured to perform a step (vi)(b) after step (vi)(a) comprising compare the further CPR performance measurement of the person with a maximum CPR performance measurement, when the further CPR performance measurement is less than the maximum CPR performance measurement go to step (vii), when the further CPR performance measurement is greater than the maximum CPR performance measurement produce the seventh prompt signal and return to step (v).

Step (vi)(b) may comprise when the further CPR performance measurement is greater than the maximum CPR performance measurement, produce the seventh prompt signal, wait for a plurality of compressions and return to step (v).

The minimum CPR performance measurement and the maximum CPR performance measurement may comprise extremes of a pre-defined acceptable range of CPR performance.

The first CPR performance measurement may comprise a measurement tolerance. The further CPR performance measurement may comprise a measurement tolerance. The measurement tolerance may be approximately 10%.

The CPR measurement system may measure compression signals during the first plurality of chest compressions by the person and during the further plurality of chest compressions by the person comprising displacement signals of the chest of the subject during the chest compressions. The displacement signals of the chest of the subject may be measured using an accelerometer positioned on the chest of the subject. The CPR assessment system may establish the first CPR performance measurement of the person by measuring and averaging an amplitude of the displacement signals over the first plurality of chest compressions by the person. The CPR assessment system may establish the further CPR performance measurement of the person by measuring and averaging an amplitude of the displacement signals over the further plurality of chest compressions by the person.

The CPR measurement system may measure compression signals during the first plurality of chest compressions by the person and during the further plurality of chest compressions by the person comprising compression-sensitive physiological signals of the subject during the chest compressions. The compression-sensitive physiological signals of the subject may comprise any of thoracic impedance signals, end tidal CO₂ signals, blood pressure signals, SpOz signals, rSOz signals, electrocardiogram signals. The CPR assessment system may establish the first CPR performance measurement of the person by measuring and averaging an amplitude of the compression-sensitive physiological signals of the subject over the first plurality of chest compressions by the person. The CPR assessment system may establish the further CPR performance measurement of the person by measuring and averaging an amplitude of the compression-sensitive physiological signals of the subject over the further plurality of chest compressions by the person.

An increase in the average amplitude of the compression-sensitive physiological signals of the subject between the first and further plurality of chest compressions by the person will indicate an improvement of the CPR performance of the person. An decrease in the average amplitude of the compression-sensitive physiological signals of the subject between the first and further plurality of chest compressions by the person will indicate a degradation of the CPR performance of the person.

The CPR measurement system may measure compression rates during the chest compressions by the person on the subject.

The feedback unit may receive the first feedback signal and issue CPR feedback in the form of a 'Start CPR and Push Hard' instruction to the person. The feedback unit may receive the second feedback signal and issue CPR feedback in the form of a 'Keep Going' instruction to the person. The feedback unit may receive the third feedback signal and issue CPR feedback in the form of a 'Push Harder' instruction to the person. The feedback unit may receive the fourth feedback signal and issue CPR feedback in the form of a 'Stop CPR' instruction to the person. The feedback unit may receive the fifth feedback signal and issue CPR feedback in the form of a 'Push Faster' instruction to the person. The feedback unit may receive the sixth feedback signal and issue CPR feedback in the form of a 'Push Slower' instruction to the person. The feedback unit may receive the seventh feedback signal and issue CPR feedback in the form of a 'Push Softer' instruction to the person.

An embodiment of the invention will now be described, by way of example only, with reference to the following drawings, in which :
Figure 1 is a schematic representation of a defibrillator according to the invention, and
Figure 2 is a flowchart of steps carried out by a CPR assessment system of the defibrillator of Figure 1.

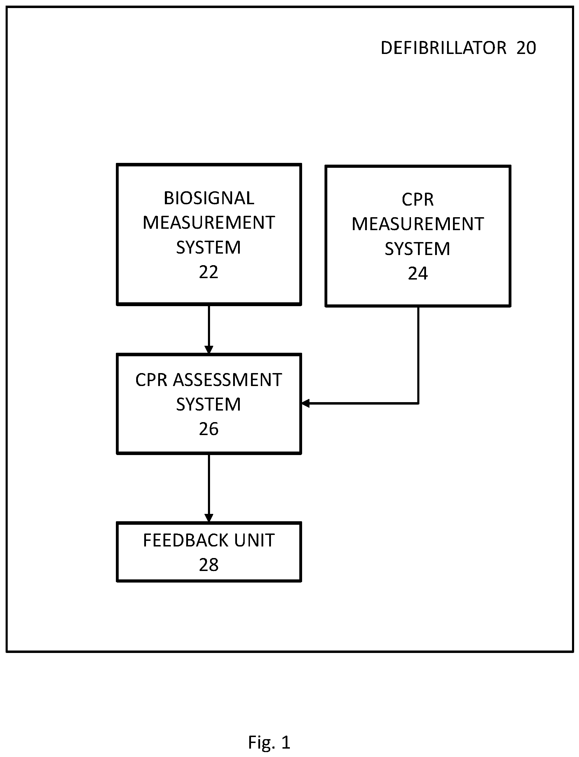

Referring to Figure 1, the defibrillator 20 comprises a biosignal measurement system 22, a CPR measurement system 24, a CPR assessment system 26 and a feedback unit 28. The defibrillator 20 assesses CPR carried out by a person (not shown) on a subject (not shown) and provides CPR feedback to the person.

It will be appreciated that the defibrillator 20 will comprise other elements such as an activation mechanism, a biosignal processing system, defibrillation shock generation circuitry, a power source and a sensing unit which is adapted to be attached to the subject.

The biosignal measurement system 22 is connected to the sensing unit and is configured to measure biosignals of the subject, in this embodiment, ECG biosignals. An algorithm uses the ECG biosignals to determine if the subject is exhibiting a condition which requires a defibrillation shock or a condition which requires CPR. When CPR is required, the biosignal measurement system 22 is configured to produce a CPR start signal. When CPR is to be ceased, the biosignal measurement system 22 is configured to produce a CPR stop signal.

In this embodiment, the CPR measurement system 24 is connected to the sensing unit and measures compression signals during chest compressions by the person. In this embodiment, the compression signals comprise compression-sensitive physiological signals consisting of thoracic impedance signals of the subject. The CPR measurement system 24 also measures compression rates during chest compressions by the person.

It will be appreciated that other compression-sensitive physiological signals can be used such as any of end tidal CO₂ signals, blood pressure signals, SpOz signals, rSOz signals, electrocardiogram signals. It will also be appreciated that the CPR measurement system could use other methods of measuring the compression signals, such as measurement of displacement signals of the chest of the subject during the chest compressions by the person.

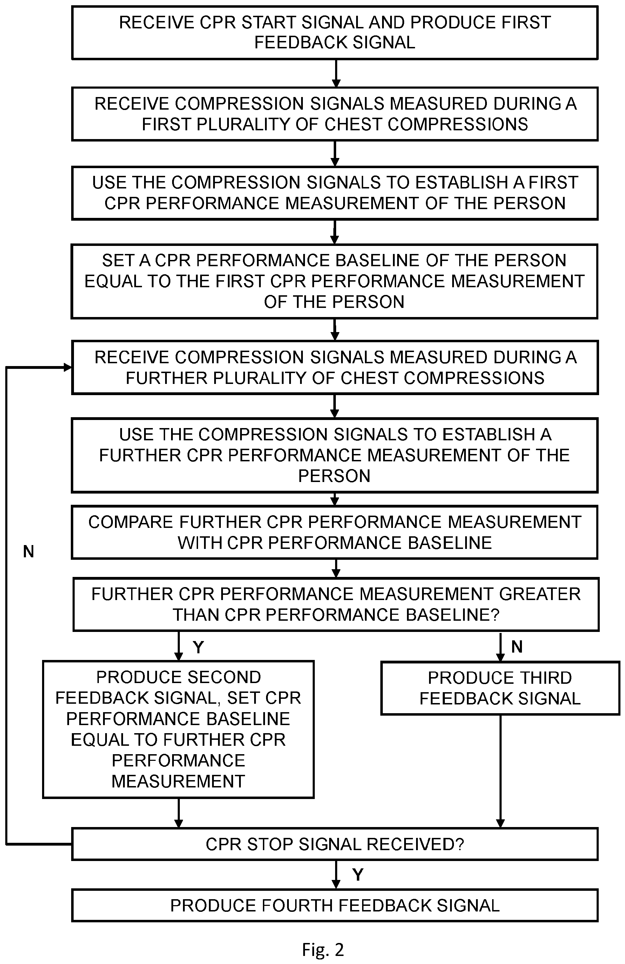

The CPR assessment system 26 is connected to the biosignal measurement system 22 to receive the CPR start signal and the CPR stop signal. On receipt of the CPR start signal, the CPR assessment system 26 receives compression signals and commences assessment of CPR performance by the person on the subject over multiple pluralities of chest compressions. This comprises performance of a number of steps, described below with reference to Figure 2. On receipt of the CPR stop signal, the CPR assessment system 26 ceases assessment of CPR performance by the person on the subject.

The CPR assessment system 26 is connected to the CPR measurement system 24 and receives compression signals indicative of chest compressions comprising the thoracic impedance signals and uses these to assess CPR performance of the person over the multiple pluralities of chest compressions carried out by the person on the subject. The CPR assessment system 26 establishes CPR performance measurements of the person by measuring and averaging amplitudes of the thoracic impedance signals over each plurality of chest compressions by the person.

During assessment of the CPR performance of the person, the CPR assessment system 26 produces various feedback signals. The feedback unit 28 is connected to the CPR assessment system 26 and is configured to receive the feedback signals and issue CPR feedback to the person.

Referring to Figure 2, the steps performed by the CPR assessment system 26 of the defibrillator 20 of Figure 1 will be described.

On receipt of the CPR start signal, the CPR assessment system 26 produces a first feedback signal. This is received by the feedback unit 28, which issues CPR feedback in the form of a 'Start CPR and Push Hard' instruction, to the person carrying out CPR on the subject.

The CPR assessment system 26 then receives -compression signals measured by the CPR measurement system 24 during a first plurality of chest compressions and uses the compression signals to establish a first CPR performance measurement of the person. A CPR performance baseline of the person is then set to be equal to the first CPR performance measurement of the person.

The CPR assessment system 26 then receives- compression signals measured by the CPR measurement system 24 during a further plurality of chest compressions and uses the compression signals to establish a further CPR performance measurement of the person.

The CPR assessment system 26 then compares the further CPR performance measurement of the person with the CPR performance baseline of the person. When the further CPR performance measurement is greater than the CPR performance baseline, the CPR assessment system 26 produces a second feedback signal and sets the CPR performance baseline equal to the further CPR performance measurement. The feedback unit 28 receives the second feedback signal and issues CPR feedback in the form of a 'Keep Going' instruction to the person carrying out CPR on the subject. This instruction is intended to encourage the person to continue applying chest compressions with the same force.

When the further CPR performance measurement of the person is less than the CPR performance baseline of the person, the CPR assessment system 26 produces a third feedback signal. The feedback unit 28 receives the third feedback signal and issues CPR feedback in the form of a 'Push Harder' instruction to the person carrying out CPR on the subject. This instruction is intended to encourage the person to apply more force in the chest compressions.

The CPR assessment system 26 then checks for receipt of the CPR stop signal from the biosignal measurement system 22. When the CPR stop signal has not been received, the CPR assessment system 26 returns to the step of receiving compression signals during a further plurality of chest compressions by the person. When the CPR stop signal is received, the CPR assessment system 26 produces a fourth feedback signal. The feedback unit 28 receives the fourth feedback signal and issues CPR feedback in the form of a 'Stop CPR' instruction to the person carrying out CPR on the subject.

The steps performed by the CPR assessment system 26 may further comprise the following.

Steps (viii) to (x) may further comprise:
(viii) when the further CPR performance measurement is greater than the CPR performance baseline, produce a second feedback signal and set the CPR performance baseline equal to the further CPR performance measurement and go to step (x)(a),
(ix) when the further CPR performance measurement is less than the CPR performance baseline, produce a third feedback signal and go to step (x)(b),
(x)(a) when the CPR stop signal is not received, return to step (v),
(x)(b) when the CPR stop signal is not received, wait for a plurality of chest compressions, return to step (v).

This allows the person performing the CPR a number of chest compressions to bring their CPR performance back up to the baseline level assessed during the first plurality of chest compressions.

Steps (viii) to (x) may further comprise:
(viii) when the further CPR performance measurement is greater than the CPR performance baseline, produce the second feedback signal and set the CPR performance baseline equal to the further CPR performance measurement and go to step (x)(a),
(ix) when the further CPR performance measurement is less than the CPR performance baseline, produce the third feedback signal and go to step (x)(b),
(x)(a) when the CPR stop signal is not received, wait for a plurality of chest compressions, return to step (v),
(x)(b) when the CPR stop signal is not received, return to step (v).

Steps (viii) to (x) may further comprise:
(viii) when the further CPR performance measurement is greater than the CPR performance baseline, produce the second feedback signal and set the CPR performance baseline equal to the further CPR performance measurement and go to step (x)(a),
(ix) when the further CPR performance measurement is less than the CPR performance baseline, produce the third feedback signal and go to step (x)(b),
(x)(a) when the CPR stop signal is not received, wait for a plurality of chest compressions, return to step (v),
(x)(b) when the CPR stop signal is not received, wait for a plurality of chest compressions, return to step (v).

Steps (ii) to (iv) may further comprise:
(ii) receive first compression signals measured during a first plurality of chest compressions and at least second compression signals measured during a second plurality of chest compressions,
(iii) use the first compression signals to establish a first CPR performance measurement of the person and use the at least second compressions signals to establish- an at least second CPR performance measurement of the person,
(iv) set a CPR performance baseline equal to the greater of the first CPR performance measurement and the at least second CPR performance measurement.

Steps (v) to (x) may further comprise:
(v)(a) set a CPR counter equal to x,
(v)(b) receive compression signals measured during a further plurality of chest compressions,
(vi) use the compression signals to establish a further CPR performance measurement,
(vii) compare the further CPR performance measurement with the CPR performance baseline,
(viii) when the further CPR performance measurement is greater than the CPR performance baseline, produce the second feedback signal, set the CPR performance baseline equal to the further CPR performance measurement and go to (x)(a),
(ix)(a) when the further CPR performance measurement is less than the CPR performance baseline, when the CPR counter is not equal to zero, produce the third feedback signal, decrease the CPR counter by 1 and go to (x)(b),
(ix)(b) when the further CPR performance measurement is less than the CPR performance baseline, when the CPR counter is equal to zero, produce the second feedback signal, decrease the CPR performance baseline and go to (x)(a),
(x)(a) when the CPR stop signal is not received, return to step (v)(a)
(x)(b) when the CPR stop signal is not received, return to step (v)(b).

Decreasing the CPR performance baseline may comprise setting the CPR performance baseline equal to the further CPR performance measurement. Decreasing the CPR performance baseline may comprise setting the CPR performance baseline equal to a greater of further CPR performance measurements.

Step (x)(a) may comprise when the CPR stop signal is not received, wait for a plurality of compressions and return to step (v)(a). Step (x)(b) may comprise when the CPR stop signal is not received, wait for a plurality of compressions and return to step (v)(b).

Step (ii) may further comprise:
(ii)(a) set a CPR counter equal to x,
(ii)(b) receive compression signals measured during a first plurality of chest compressions,
(ii)(c) receive a compression rate measured during the first plurality of chest compressions,
(ii)(d) compare the measured compression rate with a minimum required compression rate,
(ii)(e) when the measured compression rate is greater than the minimum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii).

Step (ii)(f) may comprise when the measured compression rate is less than the minimum required compression rate, when the CPR counter is not equal to zero, decrease the CPR counter by 1, produce a fifth feedback signal, wait for a plurality of compressions and go to step (ii)(b).

Step (ii) may further comprise:
(ii)(a) set a CPR counter equal to x,
(ii)(b) receive compression signals measured during a first plurality of chest compressions,
(ii)(c) receive a compression rate measured during the first plurality of chest compressions,
(ii)(d) compare the measured compression rate with a maximum required compression rate,
(ii)(e) when the measured compression rate is less than the maximum required compression rate, go to step (iii),
(ii)(f) when the measured compression rate is greater than the maximum required compression rate, when the CPR counter is not equal to zero, decrease the CPR counter by 1, produce a sixth feedback signal and go to step (ii)(b),
(ii)(g) when the measured compression rate is greater than the maximum required compression rate, when the CPR counter is equal to zero, go to step (iii).

Step (ii)(f) may comprise when the measured compression rate is greater than the maximum required compression rate, when the CPR counter is not equal to zero, decrease the CPR counter by 1, produce a sixth feedback signal, wait for a plurality of compressions and go to step (ii)(b).

Step (ii) may further comprise:
(ii)(a) set a CPR counter equal to x,
(ii)(b) receive compression signals measured during a first plurality of chest compressions,
(ii)(c) receive a compression rate measured during the first plurality of chest compressions,
(ii)(d) compare the measured compression rate with a minimum required compression rate and a maximum required compression rate,
(ii)(e) when the measured compression rate is greater than the minimum required compression rate and when the measured compression rate is less than the maximum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii), (ii)(h) when the measured compression rate is greater than the maximum required compression rate, when the CPR counter is not equal to zero, decrease the CPR counter by 1, produce a sixth feedback signal and go to step (ii)(b),
(ii)(i) when the measured compression rate is greater than the maximum required compression rate, when the CPR counter is equal to zero, go to step (iii).

Step (ii)(f) may comprise when the measured compression rate is less than the minimum required compression rate, when the CPR counter is not equal to zero, decrease the CPR counter by 1, produce a fifth feedback signal, wait for a plurality of compressions and go to step (ii)(b).

Step (ii)(h) may comprise when the measured compression rate is greater than the maximum required compression rate, when the CPR counter is not equal to zero, decrease the CPR counter by 1, produce a sixth feedback signal, wait for a plurality of compressions and go to step (ii)(b).

Step (v) may further comprise:
(v)(a) set a CPR counter equal to x,
(v)(b) receive compression signals measured during a further plurality of chest compressions,
(v)(c) receive a compression rate measured during the further plurality of chest compressions,
(v)(d) compare the measured compression rate with a minimum required compression rate,
(v)(e) when the measured compression rate is greater than the minimum required compression rate, go to step (vi),
(v)(f) when the measured compression rate is less than the minimum required compression rate, when the CPR counter is not equal to zero, decrease the CPR counter by 1, produce a fifth feedback signal and go to step (v)(b),
(v)(g) when the measured compression rate is less than the minimum required compression rate, when the CPR counter is equal to zero, go to step (vi).

Step (v)(f) may comprise when the measured compression rate is less than the minimum required compression rate, when the CPR counter is not equal to zero, decrease the CPR counter by1, produce a fifth feedback signal, wait for a plurality of compressions and go to step (v)(b).

Step (v) may further comprise:
(v)(a) set a CPR counter equal to x,
(v)(b) receive compression signals measured during a further plurality of chest compressions,
(v)(c) receive a compression rate measured during the further plurality of chest compressions,
(v)(d) compare the measured compression rate with a maximum required compression rate,
(v)(e) when the measured compression rate is less than the maximum required compression rate, go to step (vi),
(v)(f) when the measured compression rate is greater than the maximum required compression rate, when the CPR counter is not equal to zero, decrease the CPR counter by 1, produce a sixth feedback signal and go to step (v)(b),
(v)(g) when the measured compression rate is greater than the maximum required compression rate, when the CPR counter is equal to zero, go to step (vi).

Step (v)(f) may comprise when the measured compression rate is greater than the maximum required compression rate, when the CPR counter is not equal to zero, decrease the CPR counter by 1, produce a sixth feedback signal, wait for a plurality of compressions and go to step (v)(b).

Step (v) may further comprise:
(v)(a) set a CPR counter equal to x,
(v)(b) receive compression signals measured during a further plurality of chest compressions,
(v)(c) receive a compression rate measured during the further plurality of chest compressions,
(v)(d) compare the measured compression rate with a minimum required compression rate and a maximum required compression rate,
(v)(e) when the measured compression rate is greater than the minimum required compression rate and when the measured compression rate is less than the maximum required compression rate, go to step (vi),
(v)(f) when the measured compression rate is less than the minimum required compression rate, when the CPR counter is not equal to zero, decrease the CPR counter by 1, produce a fifth feedback signal and go to step (v)(b),
(v)(g) when the measured compression rate is less than the minimum required compression rate, when the CPR counter is equal to zero, go to step (vi),
(v)(h) when the measured compression rate is greater than the maximum required compression rate, when the CPR counter is not equal to zero, decrease the CPR counter by 1, produce a sixth feedback signal and go to step (v)(b),
(v)(i) when the measured compression rate is greater than the maximum required compression rate, when the CPR counter is equal to zero, go to step (vi).

Step (v)(f) may comprise when the measured compression rate is less than the minimum required compression rate, when the CPR counter is not equal to zero, decrease the CPR counter by 1, produce a fifth feedback signal, wait for a plurality of compressions and go to step (v)(b).

Step (v)(h) may comprise when the measured compression rate is greater than the maximum required compression rate, when the CPR counter is not equal to zero, decrease the CPR counter by 1, produce a sixth feedback signal, wait for a plurality of compressions and go to step (v)(b).

The minimum required compression rate may comprise approximately 100 compressions per minute. The feedback unit 28 may receive the fifth feedback signal and issue CPR feedback in the form of a 'Push Faster' instruction to the person carrying out CPR on the subject.

The maximum required compression rate may comprise approximately 120 compressions per minute. The feedback unit 28 may receive the sixth feedback signal and issue CPR feedback in the form of a 'Push Slower' instruction to the person carrying out CPR on the subject.

The CPR assessment system 26 may be configured to perform a step (iii)(a) after step (iii) comprising compare the first CPR performance measurement with a minimum CPR performance measurement, when the first CPR performance measurement is greater than the minimum CPR performance measurement go to step (iv), when the first CPR performance measurement is less than the minimum CPR performance measurement produce the third prompt signal and return to step (ii).

Step (iii)(a) may comprise when the first CPR performance measurement is less than the minimum CPR performance measurement, produce the third prompt signal, wait for a plurality of compressions and return to step (ii).

The CPR assessment system 26 may be configured to perform a step (iii)(b) after step (iii)(a) comprising compare the first CPR performance measurement with a maximum CPR performance measurement, when the first CPR performance measurement is less than the maximum CPR performance measurement go to step (iv), when the first CPR performance measurement is greater than the maximum CPR performance measurement produce a seventh prompt signal and return to step (ii).

Step (iii)(b) may comprise when the first CPR performance measurement is greater than the maximum CPR performance measurement, produce a seventh prompt signal, wait for a plurality of compressions and return to step (ii).

The feedback unit 28 may receive the seventh feedback signal and issue CPR feedback in the form of a 'Push Softer' instruction to the person carrying out CPR on the subject.

The CPR assessment system 26 may be configured to perform a step (vi)(a) after step (vi) comprising compare the further CPR performance measurement with a minimum CPR performance measurement, when the further CPR performance measurement is greater than the minimum CPR performance measurement go to step (vii), when the further CPR performance measurement is less than the minimum CPR performance measurement produce the third prompt signal and return to step (v).

Step (vi)(a) may comprise when the further CPR performance measurement is less than the minimum CPR performance measurement, produce the third prompt signal, wait for a plurality of compressions and return to step (v).

The CPR assessment system 26 may be configured to perform a step (vi)(b) after step (vi)(a) comprising compare the further CPR performance measurement with a maximum CPR performance measurement, when the further CPR performance measurement is less than the maximum CPR performance measurement go to step (vii), when the further CPR performance measurement is greater than the maximum CPR performance measurement produce the seventh prompt signal and return to step (v).

Step (vi)(b) may comprise when the further CPR performance measurement is greater than the maximum CPR performance measurement, produce a seventh prompt signal, wait for a plurality of compressions and return to step (v).

It can be seen that the CPR assessment system of the defibrillator will repeatedly compare subsequent CPR performance of the person with previous CPR performance of the person, i.e. perform a 'CPR performance self-comparison'. Adaptive CPR feedback is then provided to the person, i.e. CPR feedback adapted according to the result of the CPR performance comparison.

## Claims

1. A defibrillator (20) which assesses cardio pulmonary resuscitation (CPR) carried out by a person on a subject and provides CPR feedback to the person, comprising:
a biosignal measurement system (22) configured to measure biosignals of the subject, determine when CPR is required and produce a CPR start signal and determine and when CPR is no longer required and produce a CPR stop signal,
a CPR measurement system (24) configured to measure compression signals during CPR chest compressions by the person,
a CPR assessment system (26) connected to the biosignal measurement system (22) to receive the CPR start signal and the CPR stop signal, connected to the CPR measurement system (24) receive compression signals and configured to perform the steps:
(i) receive the CPR start signal and produce a first feedback signal,
(ii) receive compression signals measured during a first plurality of chest compressions by the person,
(iii) use the compression signals to establish a first CPR performance measurement of the person,
(iv) set a CPR performance baseline of the person equal to the first CPR performance measurement of the person,
(v) receive compression signals measured during a further plurality of chest compressions by the person,
(vi) use the compression signals to establish a further CPR performance measurement of the person,
(vii) compare the further CPR performance measurement of the person with the CPR performance baseline of the person,
(viii) when the further CPR performance measurement of the person is greater than the CPR performance baseline of the person, produce a second feedback signal and set the CPR performance baseline of the person equal to the further CPR performance measurement of the person and go to step (x),
(ix) when the further CPR performance measurement of the person is less than the CPR performance baseline of the person, produce a third feedback signal and go to step (x),
(x) when the CPR stop signal is not received, return to step (v),
(xi) when the CPR stop signal is received, produce a fourth feedback signal,
and
a feedback unit (28) connected to the CPR assessment system and configured to receive the feedback signals and issue CPR feedback to the person.

2. A defibrillator (20) according to claim 1 in which steps (viii) to (x) further comprise:
(viii) when the further CPR performance measurement is greater than the CPR performance baseline, produce the second feedback signal and set the CPR performance baseline equal to the further CPR performance measurement and go to step (x)(a),
(ix) when the further CPR performance measurement is less than the CPR performance baseline, produce the third feedback signal and go to step (x)(b),
(x)(a) when the CPR stop signal is not received, wait for a plurality of chest compressions, return to step (v),
(x)(b) when the CPR stop signal is not received, return to step (v).

3. A defibrillator (20) according to claim 1 or claim 2 in which steps (ii) to (iv) further comprise:
(ii) receive first compression signals measured during a first plurality of chest compressions and at least second compression signals measured during a second plurality of chest compressions,
(iii) use the first compression signals to establish a first CPR performance measurement and use the at least second compression signals to establish an at least second CPR performance measurement,
(iv) set a CPR performance baseline equal to the greater of the first CPR performance measurement and the at least second CPR performance measurement.

4. A defibrillator (20) according to any preceding claim in which steps (v) to (x) further comprise:
(v)(a) set a CPR counter equal to x,
(v)(b) receive compression signals measured during a further plurality of chest compressions,
(vi) use the compression signals to establish a further CPR performance measurement,
(vii) compare the further CPR performance measurement with the CPR performance baseline,
(viii) when the further CPR performance measurement is greater than the CPR performance baseline, produce the second feedback signal, set the CPR performance baseline equal to the further CPR performance measurement and go to (x)(a),
(ix)(a) when the further CPR performance measurement is less than the CPR performance baseline, when the CPR counter is not equal to zero, produce the third feedback signal, decrease the CPR counter by 1 and go to (x)(b),
(ix)(b) when the further CPR performance measurement is less than the CPR performance baseline, when the CPR counter is equal to zero, produce the second feedback signal, decrease the CPR performance baseline and go to (x)(a),
(x)(a) when the CPR stop signal is not received, return to step (v)(a)
(x)(b) when the CPR stop signal is not received, return to step (v)(b).

5. A defibrillator (20) according to any preceding claim in which step (ii) further comprises:
(ii)(a) set a CPR counter equal to x,
(ii)(b) receive compression signals measured during a first plurality of chest compressions,
(ii)(c) receive a compression rate measured during the first plurality of chest compressions,
(ii)(d) compare the measured compression rate with a minimum required compression rate,
(ii)(e) when the measured compression rate is greater than the minimum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii).

6. A defibrillator (20) according to any of claims 1 to 4 in which step (ii) further comprises:
(ii)(a) set a CPR counter equal to x,
(ii)(b) receive compression signals measured during a first plurality of chest compressions,
(ii)(c) receive a compression rate measured during the first plurality of chest compressions,
(ii)(d) compare the measured compression rate with a maximum required compression rate,
(ii)(e) when the measured compression rate is less than the maximum required compression rate, go to step (iii),
(ii)(f) when the measured compression rate is greater than the maximum required compression rate, when the CPR counter is not equal to zero, decrease the CPR counter by 1, produce a sixth feedback signal and go to step (ii)(b),
(ii)(g) when the measured compression rate is greater than the maximum required compression rate, when the CPR counter is equal to zero, go to step (iii).

7. A defibrillator (20) according to any of claims 1 to 4 in which step (ii) further comprises:
(ii)(a) set a CPR counter equal to x,
(ii)(b) receive compression signals measured during a first plurality of chest compressions,
(ii)(c) receive a compression rate measured during the first plurality of chest compressions,
(ii)(d) compare the measured compression rate with a minimum required compression rate and a maximum required compression rate,
(ii)(e) when the measured compression rate is greater than the minimum required compression rate and when the measured compression rate is less than the maximum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii), (ii)(h) when the measured compression rate is greater than the maximum required compression rate, when the CPR counter is not equal to zero, decrease the CPR counter by 1, produce a sixth feedback signal and go to step (ii)(b),
(ii)(i) when the measured compression rate is greater than the maximum required compression rate, when the CPR counter is equal to zero, go to step (iii).

8. A defibrillator (20) according to any preceding claim in which step (v) further comprises:
(v)(a) set a CPR counter equal to x,
(v)(b) receive compression signals measured during a further plurality of chest compressions,
(v)(c) receive a compression rate measured during the further plurality of chest compressions,
(v)(d) compare the measured compression rate with a minimum required compression rate,
(v)(e) when the measured compression rate is greater than the minimum required compression rate, go to step (vi),
(v)(f) when the measured compression rate is less than the minimum required compression rate, when the CPR counter is not equal to zero, decrease the CPR counter by 1, produce a fifth feedback signal and go to step (v)(b),
(v)(g) when the measured compression rate is less than the minimum required compression rate, when the CPR counter is equal to zero, go to step (vi).

9. A defibrillator (20) according to any of claims 1 to 7 in which step (v) further comprises:
(v)(a) set a CPR counter equal to x,
(v)(b) receive compression signals measured during a further plurality of chest compressions,
(v)(c) receive a compression rate measured during the further plurality of chest compressions,
(v)(d) compare the measured compression rate with a maximum required compression rate,
(v)(e) when the measured compression rate is less than the maximum required compression rate, go to step (vi),
(v)(f) when the measured compression rate is greater than the maximum required compression rate, when the CPR counter is not equal to zero, decrease the CPR counter by 1, produce a sixth feedback signal and go to step (v)(b),
(v)(g) when the measured compression rate is greater than the maximum required compression rate, when the CPR counter is equal to zero, go to step (vi).

10. A defibrillator (20) according to any of claims 1 to 7 in which step (v) further comprises:
(v)(a) set a CPR counter equal to x,
(v)(b) receive compression signals measured during a further plurality of chest compressions,
(v)(c) receive a compression rate measured during the further plurality of chest compressions,
(v)(d) compare the measured compression rate with a minimum required compression rate and a maximum required compression rate,
(v)(e) when the measured compression rate is greater than the minimum required compression rate and when the measured compression rate is less than the maximum required compression rate, go to step (vi),
(v)(f) when the measured compression rate is less than the minimum required compression rate, when the CPR counter is not equal to zero, decrease the CPR counter by 1, produce a fifth feedback signal and go to step (v)(b),
(v)(g) when the measured compression rate is less than the minimum required compression rate, when the CPR counter is equal to zero, go to step (vi),
(v)(h) when the measured compression rate is greater than the maximum required compression rate, when the CPR counter is not equal to zero, decrease the CPR counter by 1, produce a sixth feedback signal and go to step (v)(b),
(v)(i) when the measured compression rate is greater than the maximum required compression rate, when the CPR counter is equal to zero, go to step (vi).

11. A defibrillator (20) according to any preceding claim in which the CPR assessment system (26) is configured to perform a step (iii)(a) after step (iii) comprising compare the first CPR performance measurement with a minimum CPR performance measurement, when the first CPR performance measurement is greater than the minimum CPR performance measurement go to step (iv), when the first CPR performance measurement is less than the minimum CPR performance measurement produce the third prompt signal and return to step (ii).

12. A defibrillator (20) according to claim 11 in which the CPR assessment system (26) is configured to perform a step (iii)(b) after step (iii)(a) comprising compare the first CPR performance measurement with a maximum CPR performance measurement, when the first CPR performance measurement is less than the maximum CPR performance measurement go to step (iv), when the first CPR performance measurement is greater than the maximum CPR performance measurement produce a seventh prompt signal and return to step (ii).

13. A defibrillator (20) according to any preceding claim in which the CPR assessment system (26) is configured to perform a step (vi)(a) after step (vi) comprising compare the further CPR performance measurement with a minimum CPR performance measurement, when the further CPR performance measurement is greater than the minimum CPR performance measurement go to step (vii), when the further CPR performance measurement is less than the minimum CPR performance measurement produce the third prompt signal and return to step (v).

14. A defibrillator (20) according to claim 13 in which the CPR assessment system (26) is configured to perform a step (vi)(b) after step (vi)(a) comprising compare the further CPR performance measurement with a maximum CPR performance measurement, when the further CPR performance measurement is less than the maximum CPR performance measurement go to step (vii), when the further CPR performance measurement is greater than the maximum CPR performance measurement produce the seventh prompt signal and return to step (v).

15. A defibrillator (20) according to any preceding claim in which the CPR measurement system (24) measures compression signals during the first plurality of chest compressions and during the further plurality of chest compressions comprising compression-sensitive physiological signals of the subject during the chest compressions.

## Patentansprüche

1. Defibrillator (20), der die von einer Person an einem Subjekt durchgeführte Herz-Lungen-Wiederbelebung (HLW) beurteilt und der Person ein HLW-Feedback bereitstellt, umfassend:
ein Biosignal-Messsystem (22), das konfiguriert ist, um Biosignale des Subjekts zu messen, zu bestimmen, wann eine HLW erforderlich ist, und ein HLW-Startsignal zu erzeugen, und zu bestimmen, und wann HLW nicht mehr erforderlich ist, und ein HLW-Stoppsignal zu erzeugen,
ein HLW-Messsystem (24), das konfiguriert ist, um Kompressionssignale während HLW-Brustkompressionen durch die Person zu messen,
ein HLW-Beurteilungssystem (26), das mit dem Biosignal-Messsystem (22) verbunden ist, um das HLW-Startsignal und das HLW-Stoppsignal zu empfangen, das mit dem HLW-Messsystem (24) verbunden ist, um Kompressionssignale zu empfangen und konfiguriert ist, um die folgenden Schritte auszuführen:
(i) Empfangen des HLW-Startsignals und Erzeugen eines ersten Feedbacksignals,
(ii) Empfangen von Kompressionssignalen, die während einer ersten Vielzahl von Brustkompressionen durch die Person gemessen werden,
(iii) Verwenden der Kompressionssignale, um eine erste HLW-Leistungsmessung der Person zu erstellen,
(iv) Festlegen eines HLW-Leistung-Ausgangswerts der Person, die der ersten HLW-Leistungsmessung der Person entspricht,
(v) Empfangen von Kompressionssignalen, die während einer weiteren Vielzahl von Brustkompressionen durch die Person gemessen werden,
(vi) Verwenden der Kompressionssignale, um eine weitere HLW-Leistungsmessung der Person zu erstellen,
(vii) Vergleichen der weiteren HLW-Leistungsmessung der Person mit dem HLW-Leistung-Ausgangswert der Person,
(viii) wenn die weitere HLW-Leistungsmessung der Person größer ist als der HLW-Leistung-Ausgangswert der Person, Erzeugen eines zweiten Feedbacksignal und Festlegen des HLW-Leistung-Ausgangswerts der Person entsprechend der weiteren HLW-Leistungsmessung der Person und Gehen zu Schritt (x),
(ix) wenn die weitere HLW-Leistungsmessung der Person geringer ist als der HLW-Leistung-Ausgangswert der Person, Erzeugen eines dritten Feedbacksignals und Gehen zu Schritt (x),
(x) wenn das HLW-Stoppsignal nicht empfangen wird, Zurückkehren zu Schritt (v),
(xi) wenn das HLW-Stoppsignal empfangen wird, Erzeugen eines vierten Feedbacksignals, und
eine Feedback-Einheit (28), die mit dem HLW-Beurteilungssystem verbunden und konfiguriert ist, um die Feedbacksignale zu empfangen und ein HLW-Feedback an die Person auszugeben.

2. Defibrillator (20) nach Anspruch 1, wobei die Schritte (viii) bis (x) ferner Folgendes umfassen:
(viii) wenn die weitere HLW-Leistungsmessung größer ist als der HLW-Leistung-Ausgangswert, Erzeugen des zweiten Feedbacksignals und Festlegen des HLW-Leistung-Ausgangswerts entsprechend der weiteren HLW-Leistungsmessung und Gehen zu Schritt (x)(a),
(ix) wenn die weitere HLW-Leistungsmessung geringer als der HLW-Leistung-Ausgangswert ist, Erzeugen des dritten Feedbacksignals und Gehen zu Schritt (x)(b),
(x)(a) wenn das HLW-Stoppsignal nicht empfangen wird, Warten während einer Vielzahl von Brustkompressionen, Zurückkehren zu Schritt (v),
(x)(b) wenn das HLW-Stoppsignal nicht empfangen wird, Zurückkehren zu Schritt (v).

3. Defibrillator (20) nach Anspruch 1 oder Anspruch 2, wobei die Schritte (ii) bis (iv) ferner Folgendes umfassen:
(ii) Empfangen erster Kompressionssignale, die während einer ersten Vielzahl von Brustkompressionen gemessen werden, und mindestens zweiter Kompressionssignale, die während einer zweiten Vielzahl von Brustkompressionen gemessen werden,
(iii) Verwenden der ersten Kompressionssignale, um eine erste HLW-Leistungsmessung zu erstellen, und Verwenden der mindestens zweiten Kompressionssignale, um eine mindestens zweite HLW-Leistungsmessung zu erstellen,
(iv) Festlegen eines HLW-Leistung-Ausgangswerts, der dem größeren Wert aus der ersten HLW-Leistungsmessung und der mindestens zweiten HLW-Leistungsmessung entspricht.

4. Defibrillator (20) nach einem vorhergehenden Anspruch, wobei die Schritte (v) bis (x) ferner Folgendes umfassen:
(v)(a) Festlegen eines HLW-Zählers auf x,
(v)(b) Empfangen von Kompressionssignalen, die während einer weiteren Vielzahl von Brustkompressionen gemessen werden,
(vi) Verwenden der Kompressionssignale, um eine weitere HLW-Leistungsmessung zu erstellen,
(vii) Vergleichen der weiteren HLW-Leistungsmessung mit dem HLW-Leistung-Ausgangswert,
(viii) wenn die weitere HLW-Leistungsmessung größer ist als der HLW-Leistung-Ausgangswert, Erzeugen des zweiten Feedbacksignals, Festlegen des HLW-Leistung-Ausgangswerts entsprechend der weiteren HLW-Leistungsmessung und Gehen zu (x)(a),
(ix)(a) wenn die weitere HLW-Leistungsmessung geringer ist als der HLW-Leistung-Ausgangswert, wenn der HLW-Zähler nicht gleich Null ist, Erzeugen des dritten Feedbacksignals, Verringern des HLW-Zählers um 1 und Gehen zu (x)(b),
(ix)(b) wenn die weitere HLW-Leistungsmessung geringer ist als der HLW-Leistung-Ausgangswert, wenn der HLW-Zähler gleich Null ist, Erzeugen des zweiten Feedbacksignals, Verringern des HLW-Leistung-Ausgangswerts und Gehen zu (x)(a),
(x)(a) wenn das HLW-Stoppsignal nicht empfangen wird, Zurückkehren zu Schritt (v)(a),
(x)(b) wenn das HLW-Stoppsignal nicht empfangen wird, Zurückkehren zu Schritt (v)(b).

5. Defibrillator (20) nach einem vorhergehenden Anspruch, wobei Schritt (ii) ferner Folgendes umfasst:
(ii)(a) Festlegen eines HLW-Zählers auf x,
(ii)(b) Empfangen von Kompressionssignalen, die während einer ersten Vielzahl von Brustkompressionen gemessen werden,
(ii)(c) Empfangen einer Kompressionsrate, die während der ersten Vielzahl von Brustkompressionen gemessen wird,
(ii)(d) Vergleichen der gemessenen Kompressionsrate mit einer erforderlichen minimalen Kompressionsrate,
(ii)(e) wenn die gemessene Kompressionsrate größer ist als die erforderliche minimale Kompressionsrate, Gehen zu Schritt (iii),
(ii)(f) wenn die gemessene Kompressionsrate geringer ist als die erforderliche minimale Kompressionsrate, wenn der HLW-Zähler nicht gleich Null ist, Verringern des HLW-Zählers um 1, Erzeugen eines fünften Feedbacksignals und Gehen zu Schritt (ii)(b),
(ii)(g) wenn die gemessene Kompressionsrate geringer ist als die erforderliche minimale Kompressionsrate, wenn der HLW-Zähler gleich Null ist, Gehen zu Schritt (iii).

6. Defibrillator (20) nach einem der Ansprüche 1 bis 4, wobei Schritt (ii) ferner Folgendes umfasst:
(ii)(a) Festlegen eines HLW-Zählers auf x,
(ii)(b) Empfangen von Kompressionssignalen, die während einer ersten Vielzahl von Brustkompressionen gemessen werden,
(ii)(c) Empfangen einer Kompressionsrate, die während der ersten Vielzahl von Brustkompressionen gemessen wird,
(ii)(d) Vergleichen der gemessenen Kompressionsrate mit einer erforderlichen maximalen Kompressionsrate,
(ii)(e) wenn die gemessene Kompressionsrate geringer ist als die erforderliche maximale Kompressionsrate, Gehen zu Schritt (iii),
(ii)(f) wenn die gemessene Kompressionsrate größer ist als die erforderliche maximale Kompressionsrate, wenn der HLW-Zähler nicht gleich Null ist, Verringern des HLW-Zählers um 1, Erzeugen eines sechsten Feedbacksignals und Gehen zu Schritt (ii)(b),
(ii)(g) wenn die gemessene Kompressionsrate größer ist als die erforderliche maximale Kompressionsrate, wenn der HLW-Zähler gleich Null ist, Gehen zu Schritt (iii).

7. Defibrillator (20) nach einem der Ansprüche 1 bis 4, wobei Schritt (ii) ferner Folgendes umfasst:
(ii)(a) Festlegen eines HLW-Zählers auf x,
(ii)(b) Empfangen von Kompressionssignalen, die während einer ersten Vielzahl von Brustkompressionen gemessen werden,
(ii)(c) Empfangen einer Kompressionsrate, die während der ersten Vielzahl von Brustkompressionen gemessen wird,
(ii)(d) Vergleichen der gemessenen Kompressionsrate mit einer erforderlichen minimalen Kompressionsrate und einer erforderlichen maximalen Kompressionsrate,
(ii)(e) wenn die gemessene Kompressionsrate größer ist als die erforderliche minimale Kompressionsrate und wenn die gemessene Kompressionsrate geringer ist als die erforderliche maximale Kompressionsrate, Gehen zu Schritt (iii),
(ii)(f) wenn die gemessene Kompressionsrate geringer ist als die erforderliche minimale Kompressionsrate, wenn der HLW-Zähler nicht gleich Null ist, Verringern des HLW-Zählers um 1, Erzeugen eines fünften Feedbacksignals und Gehen zu Schritt (ii)(b),
(ii)(g) wenn die gemessene Kompressionsrate geringer ist als die erforderliche minimale Kompressionsrate, wenn der HLW-Zähler gleich Null ist, Gehen zu Schritt (iii),
(ii)(h) wenn die gemessene Kompressionsrate größer ist als die erforderliche maximale Kompressionsrate, wenn der HLW-Zähler nicht gleich Null ist, Verringern des HLW-Zählers um 1, Erzeugen eines sechsten Feedbacksignals und Gehen zu Schritt (ii)(b),
(ii)(i) wenn die gemessene Kompressionsrate größer ist als die erforderliche maximale Kompressionsrate, wenn der HLW-Zähler gleich Null ist, Gehen zu Schritt (iii).

8. Defibrillator (20) nach einem vorhergehenden Anspruch, wobei Schritt (v) ferner Folgendes umfasst:
(v)(a) Festlegen eines HLW-Zählers auf x,
(v)(b) Empfangen von Kompressionssignalen, die während einer weiteren Vielzahl von Brustkompressionen gemessen werden,
(v)(c) Empfangen einer Kompressionsrate, die während der weiteren Vielzahl von Brustkompressionen gemessen wird,
(v)(d) Vergleichen der gemessenen Kompressionsrate mit einer erforderlichen minimalen Kompressionsrate,
(v)(e) wenn die gemessene Kompressionsrate größer ist als die erforderliche minimale Kompressionsrate, Gehen zu Schritt (vi),
(v)(f) wenn die gemessene Kompressionsrate geringer ist als die erforderliche minimale Kompressionsrate, wenn der HLW-Zähler nicht gleich Null ist, Verringern des HLW-Zählers um 1, Erzeugen eines fünften Feedbacksignals und Gehen zu Schritt (v)(b),
(v)(g) wenn die gemessene Kompressionsrate geringer ist als die erforderliche minimale Kompressionsrate, wenn der HLW-Zähler gleich Null ist, Gehen zu Schritt (vi).

9. Defibrillator (20) nach einem der Ansprüche 1 bis 7, wobei Schritt (v) ferner Folgendes umfasst:
(v)(a) Festlegen eines HLW-Zählers auf x,
(v)(b) Empfangen von Kompressionssignalen, die während einer weiteren Vielzahl von Brustkompressionen gemessen werden,
(v)(c) Empfangen einer Kompressionsrate, die während der weiteren Vielzahl von Brustkompressionen gemessen wird,
(v)(d) Vergleichen der gemessenen Kompressionsrate mit einer erforderlichen maximalen Kompressionsrate,
(v)(e) wenn die gemessene Kompressionsrate geringer ist als die erforderliche maximale Kompressionsrate, Gehen zu Schritt (vi),
(v)(f) wenn die gemessene Kompressionsrate größer ist als die erforderliche maximale Kompressionsrate, wenn der HLW-Zähler nicht gleich Null ist, Verringern des HLW-Zählers um 1, Erzeugen eines sechsten Feedbacksignals und Gehen zu Schritt (v)(b),
(v)(g) wenn die gemessene Kompressionsrate größer ist als die erforderliche maximale Kompressionsrate, wenn der HLW-Zähler gleich Null ist, Gehen zu Schritt (vi).

10. Defibrillator (20) nach einem der Ansprüche 1 bis 7, wobei Schritt (v) ferner Folgendes umfasst:
(v)(a) Festlegen eines HLW-Zählers auf x,
(v)(b) Empfangen von Kompressionssignalen, die während einer weiteren Vielzahl von Brustkompressionen gemessen werden,
(v)(c) Empfangen einer Kompressionsrate, die während der weiteren Vielzahl von Brustkompressionen gemessen wird,
(v)(d) Vergleichen der gemessenen Kompressionsrate mit einer erforderlichen minimalen Kompressionsrate und einer erforderlichen maximalen Kompressionsrate,
(v)(e) wenn die gemessene Kompressionsrate größer ist als die erforderliche minimale Kompressionsrate und wenn die gemessene Kompressionsrate geringer ist als die erforderliche maximale Kompressionsrate, Gehen zu Schritt (vi),
(v)(f) wenn die gemessene Kompressionsrate geringer ist als die erforderliche minimale Kompressionsrate, wenn der HLW-Zähler nicht gleich Null ist, Verringern des HLW-Zählers um 1, Erzeugen eines fünften Feedbacksignals und Gehen zu Schritt (v)(b),
(v)(g) wenn die gemessene Kompressionsrate geringer ist als die erforderliche minimale Kompressionsrate, wenn der HLW-Zähler gleich Null ist, Gehen zu Schritt (vi),
(v)(h) wenn die gemessene Kompressionsrate größer ist als die erforderliche maximale Kompressionsrate, wenn der HLW-Zähler nicht gleich Null ist, Verringern des HLW-Zählers um 1, Erzeugen eines sechsten Feedbacksignals und Gehen zu Schritt (v)(b),
(v)(i) wenn die gemessene Kompressionsrate größer ist als die erforderliche maximale Kompressionsrate, wenn der HLW-Zähler gleich Null ist, Gehen zu Schritt (vi).

11. Defibrillator (20) nach einem vorhergehenden Anspruch, wobei das HLW-Beurteilungssystem (26) konfiguriert ist, um nach Schritt (iii) einen Schritt (iii)(a) auszuführen, umfassend ein Vergleichen der ersten HLW-Leistungsmessung mit einer minimalen HLW-Leistungsmessung, wenn die erste HLW-Leistungsmessung größer ist als die minimale HLW-Leistungsmessung, Gehen zu Schritt (iv), wenn die erste HLW-Leistungsmessung geringer ist als die minimale HLW-Leistungsmessung, Erzeugen des dritten Aufforderungssignals und Zurückkehren zu Schritt (ii).

12. Defibrillator (20) nach Anspruch 11, wobei das HLW-Beurteilungssystem (26) konfiguriert ist, um nach Schritt (iii)(a) einen Schritt (iii)(b) auszuführen, umfassend ein Vergleichen der ersten HLW-Leistungsmessung mit einer maximalen HLW-Leistungsmessung, wenn die erste HLW-Leistungsmessung geringer ist als die maximale HLW-Leistungsmessung, Gehen zu Schritt (iv), wenn die erste HLW-Leistungsmessung größer ist als die maximale HLW-Leistungsmessung, Erzeugen eines siebten Aufforderungssignals und Zurückkehren zu Schritt (ii).

13. Defibrillator (20) nach einem vorhergehenden Anspruch, wobei das HLW-Beurteilungssystem (26) konfiguriert ist, um nach Schritt (vi) einen Schritt (vi)(a) auszuführen, umfassend ein Vergleichen der weiteren HLW-Leistungsmessung mit einer minimalen HLW-Leistungsmessung, wenn die weitere HLW-Leistungsmessung größer ist als die minimale HLW-Leistungsmessung, Gehen zu Schritt (vii), wenn die weitere HLW-Leistungsmessung geringer ist als die minimale HLW-Leistungsmessung, Erzeugen des dritten Aufforderungssignals und Zurückkehren zu Schritt (v).

14. Defibrillator (20) nach Anspruch 13, wobei das HLW-Beurteilungssystem (26) konfiguriert ist, um nach Schritt (vi)(a) einen Schritt (vi)(b) auszuführen, umfassend ein Vergleichen der weiteren HLW-Leistungsmessung mit einer maximalen HLW-Leistungsmessung, wenn die weitere HLW-Leistungsmessung geringer ist als die maximale HLW-Leistungsmessung, Gehen zu Schritt (vii), wenn die weitere HLW-Leistungsmessung größer ist als die maximale HLW-Leistungsmessung, Erzeugen des siebten Aufforderungssignals und Zurückkehren zu Schritt (v).

15. Defibrillator (20) nach einem vorhergehenden Anspruch, wobei das HLW-Messsystem (24) Kompressionssignale während der ersten Vielzahl von Brustkompressionen und während der weiteren Vielzahl von Brustkompressionen misst, umfassend kompressionssensitive physiologische Signale des Subjekts während der Brustkompressionen.

## Revendications

1. Défibrillateur (20) qui évalue la réanimation cardio-pulmonaire (RCP) effectuée par une personne sur un sujet et fournit une rétroaction de RCP à la personne, comprenant :
un système de mesure de biosignaux (22) conçu pour mesurer les biosignaux du sujet, déterminer quand la RCP est requise et produire un signal de démarrage de RCP et déterminer et quand la RCP n'est plus nécessaire et produire un signal d'arrêt de RCP,
un système de mesure de RCP (24) conçu pour mesurer des signaux de compression pendant les compressions thoraciques de RCP par la personne,
un système d'évaluation de RCP (26) branché au système de mesure de biosignaux (22) destiné à recevoir le signal de démarrage de RCP et le signal d'arrêt de RCP, branché au système de mesure de RCP (24) recevant des signaux de compression et conçu pour effectuer les étapes :
(i) réception du signal de démarrage de RCP et production d'un premier signal de rétroaction,
(ii) réception de signaux de compression mesurés pendant une première pluralité de compressions thoraciques par la personne,
(iii) utilisation des signaux de compression pour établir une première mesure de performance de RCP de la personne,
(iv) établissement d'une référence de performance de RCP de la personne égale à la première mesure de performance de RCP de la personne,
(v) réception de signaux de compression mesurés pendant une pluralité supplémentaire de compressions thoraciques par la personne,
(vi) utilisation des signaux de compression pour établir une mesure supplémentaire de performance de RCP de la personne,
(vii) comparaison de la mesure supplémentaire de performance de RCP de la personne à la référence de performance de RCP de la personne,
(viii) lorsque la mesure supplémentaire de performance de RCP de la personne est supérieure à la référence de performance de RCP de la personne, production d'un deuxième signal de rétroaction et définition de la référence de performance de RCP de la personne égale à la mesure supplémentaire de performance de RCP de la personne et passage à l'étape (x),
(ix) lorsque la mesure supplémentaire de performance de RCP de la personne est inférieure à la référence de performance de RCP de la personne, production d'un troisième signal de rétroaction et passage à l'étape (x),
(x) lorsque le signal d'arrêt de RCP n'est pas reçu, retour à l'étape (v),
(xi) lorsque le signal d'arrêt de RCP est reçu, production d'un quatrième signal de rétroaction, et
une unité de rétroaction (28) branchée au système d'évaluation de RCP et conçue pour recevoir les signaux de rétroaction et émettre une rétroaction de RCP à la personne.

2. Défibrillateur (20) selon la revendication 1, lesdites étapes (viii) à (x) comprenant en outre :
(viii) lorsque la mesure supplémentaire de performance de RCP est supérieure à la référence de performance de RCP, la production du deuxième signal de rétroaction et la définition de la référence de performance de RCP égale à la mesure supplémentaire de performance de RCP et le passage à l'étape (x)(a),
(ix) lorsque la mesure supplémentaire de performance de RCP est inférieure à la référence de performance de RCP, la production du troisième signal de rétroaction et le passage à l'étape (x)(b),
(x)(a) lorsque le signal d'arrêt de RCP n'est pas reçu, l'attente d'une pluralité de compressions thoraciques, le retour à l'étape (v),
(x)(b) lorsque le signal d'arrêt de RCP n'est pas reçu, le retour à l'étape (v).

3. Défibrillateur (20) selon la revendication 1 ou la revendication 2, lesdites étapes (ii) à (iv) comprenant en outre :
(ii) la réception de premiers signaux de compression mesurés pendant une première pluralité de compressions thoraciques et au moins des seconds signaux de compression mesurés pendant une seconde pluralité de compressions thoraciques,
(iii) l'utilisation des premiers signaux de compression pour établir une première mesure de performance de RCP et l'utilisation desdits au moins seconds signaux de compression pour établir au moins une seconde mesure de performance de RCP,
(iv) la définition d'une référence de performance de RCP égale à la plus élevée parmi la première mesure de performance de RCP et ladite au moins une seconde mesure de performance de RCP.

4. Défibrillateur (20) selon l'une quelconque des revendications précédentes, lesdites étapes (v) à (x) comprenant en outre :
(v)(a) le réglage d'un compteur de RCP égal à x,
(v)(b) la réception de signaux de compression mesurés pendant une pluralité supplémentaire de compressions thoraciques,
(vi) l'utilisation des signaux de compression pour établir une mesure supplémentaire de performance de RCP,
(vii) la comparaison de la mesure supplémentaire de performance de RCP à la référence de performance de RCP,
(viii) lorsque la mesure supplémentaire de performance de RCP est supérieure à la référence de performance de RCP, la production du deuxième signal de rétroaction, la définition de la référence de performance de RCP égale à la mesure supplémentaire de performance de RCP et le passage à (x)(a),
(ix)(a) lorsque la mesure supplémentaire de performance de RCP est inférieure à la référence de performance de RCP, lorsque le compteur de RCP n'est pas égal à zéro, la production du troisième signal de rétroaction, la diminution du compteur de RCP de 1 et le passage à (x)(b),
(ix)(b) lorsque la mesure supplémentaire de performance de RCP est inférieure à la référence de performance de RCP, lorsque le compteur de RCP est égal à zéro, la production du deuxième signal de rétroaction, la diminution de la référence de performance de RCP et le passage à (x)(a),
(x)(a) lorsque le signal d'arrêt de RCP n'est pas reçu, le retour à l'étape (v)(a),
(x)(b) lorsque le signal d'arrêt de RCP n'est pas reçu, le retour à l'étape (v)(b).

5. Défibrillateur (20) selon l'une quelconque des revendications précédentes, ladite étape (ii) comprenant en outre :
(ii)(a) le réglage d'un compteur de RCP égal à x,
(ii)(b) la réception de signaux de compression mesurés pendant une première pluralité de compressions thoraciques,
(ii)(c) la réception d'un taux de compression mesuré pendant la première pluralité de compressions thoraciques,
(ii)(d) la comparaison du taux de compression mesuré à un taux de compression minimal requis,
(ii)(e) lorsque le taux de compression mesuré est supérieur au taux de compression minimal requis, le passage à l'étape (iii),
(ii)(f) lorsque le taux de compression mesuré est inférieur au taux de compression minimal requis, lorsque le compteur de RCP n'est pas égal à zéro, la diminution du compteur de RCP de 1, la production d'un cinquième signal de rétroaction et le passage à l'étape (ii)(b),
(ii)(g) lorsque le taux de compression mesuré est inférieur au taux de compression minimal requis, lorsque le compteur de RCP est égal à zéro, le passage à l'étape (iii).

6. Défibrillateur (20) selon l'une quelconque des revendications 1 à 4, ladite étape (ii) comprenant en outre :
(ii)(a) le réglage d'un compteur de RCP égal à x,
(ii)(b) la réception de signaux de compression mesurés pendant une première pluralité de compressions thoraciques,
(ii)(c) la réception d'un taux de compression mesuré pendant la première pluralité de compressions thoraciques,
(ii)(d) la comparaison du taux de compression mesuré à un taux de compression maximal requis,
(ii)(e) lorsque le taux de compression mesuré est inférieur au taux de compression maximal requis, le passage à l'étape (iii),
(ii)(f) lorsque le taux de compression mesuré est supérieur au taux de compression maximal requis, lorsque le compteur de RCP n'est pas égal à zéro, la diminution du compteur de RCP de 1, la production d'un sixième signal de rétroaction et le passage à l'étape (ii)(b),
(ii)(g) lorsque le taux de compression mesuré est supérieur au taux de compression maximal requis, lorsque le compteur de RCP est égal à zéro, le passage à l'étape (iii).

7. Défibrillateur (20) selon l'une quelconque des revendications 1 à 4, ladite étape (ii) comprenant en outre :
(ii)(a) le réglage d'un compteur de RCP égal à x,
(ii)(b) la réception de signaux de compression mesurés pendant une première pluralité de compressions thoraciques,
(ii)(c) la réception d'un taux de compression mesuré pendant la première pluralité de compressions thoraciques,
(ii)(d) la comparaison du taux de compression mesuré à un taux de compression minimal requis et à un taux de compression maximal requis,
(ii)(e) lorsque le taux de compression mesuré est supérieur au taux de compression minimal requis et lorsque le taux de compression mesuré est inférieur au taux de compression maximal requis, le passage à l'étape (iii),
(ii)(f) lorsque le taux de compression mesuré est inférieur au taux de compression minimal requis, lorsque le compteur de RCP n'est pas égal à zéro, la diminution du compteur de RCP de 1, la production d'un cinquième signal de rétroaction et le passage à l'étape (ii)(b),
(ii)(g) lorsque le taux de compression mesuré est inférieur au taux de compression minimal requis, lorsque le compteur de RCP est égal à zéro, le passage à l'étape (iii),
(ii)(h) lorsque le taux de compression mesuré est supérieur au taux de compression maximal requis, lorsque le compteur de RCP n'est pas égal à zéro, la diminution du compteur de RCP de 1, la production d'un sixième signal de rétroaction et le passage à l'étape (ii)(b),
(ii)(i) lorsque le taux de compression mesuré est supérieur au taux de compression maximal requis, lorsque le compteur de RCP est égal à zéro, le passage à l'étape (iii).

8. Défibrillateur (20) selon l'une quelconque des revendications précédentes, ladite étape (v) comprenant en outre :
(v)(a) le réglage d'un compteur de RCP égal à x,
(v)(b) la réception de signaux de compression mesurés pendant une pluralité supplémentaire de compressions thoraciques,
(v)(c) la réception d'un taux de compression mesuré pendant la pluralité supplémentaire de compressions thoraciques,
(v)(d) la comparaison du taux de compression mesuré à un taux de compression minimal requis,
(v)(e) lorsque le taux de compression mesuré est supérieur au taux de compression minimal requis, le passage à l'étape (vi),
(v)(f) lorsque le taux de compression mesuré est inférieur au taux de compression minimal requis, lorsque le compteur de RCP n'est pas égal à zéro, la diminution du compteur de RCP de 1, la production d'un cinquième signal de retour et le passage à l'étape (v)(b),
(v)(g) lorsque le taux de compression mesuré est inférieur au taux de compression minimal requis, lorsque le compteur de RCP est égal à zéro, le passage à l'étape (vi).

9. Défibrillateur (20) selon l'une quelconque des revendications 1 à 7, ladite étape (v) comprenant en outre :
(v)(a) le réglage d'un compteur de RCP égal à x,
(v)(b) la réception de signaux de compression mesurés pendant une pluralité supplémentaire de compressions thoraciques,
(v)(c) la réception d'un taux de compression mesuré pendant la pluralité supplémentaire de compressions thoraciques,
(v)(d) la comparaison du taux de compression mesuré à un taux de compression maximal requis,
(v)(e) lorsque le taux de compression mesuré est inférieur au taux de compression maximal requis, le passage à l'étape (vi),
(v)(f) lorsque le taux de compression mesuré est supérieur au taux de compression maximal requis, lorsque le compteur de RCP n'est pas égal à zéro, la diminution du compteur de RCP de 1, la production d'un sixième signal de rétroaction et le passage à l'étape (v)(b),
(v)(g) lorsque le taux de compression mesuré est supérieur au taux de compression maximal requis, lorsque le compteur de RCP est égal à zéro, le passage à l'étape (vi).

10. Défibrillateur (20) selon l'une quelconque des revendications 1 à 7, ladite étape (v) comprenant en outre :
(v)(a) le réglage d'un compteur de RCP égal à x,
(v)(b) la réception de signaux de compression mesurés pendant une pluralité supplémentaire de compressions thoraciques,
(v)(c) la réception d'un taux de compression mesuré pendant la pluralité supplémentaire de compressions thoraciques,
(v)(d) la comparaison du taux de compression mesuré à un taux de compression minimal requis et à un taux de compression maximal requis,
(v)(e) lorsque le taux de compression mesuré est supérieur au taux de compression minimal requis et lorsque le taux de compression mesuré est inférieur au taux de compression maximal requis, le passage à l'étape (vi),
(v)(f) lorsque le taux de compression mesuré est inférieur au taux de compression minimal requis, lorsque le compteur de RCP n'est pas égal à zéro, la diminution du compteur de RCP de 1, la production d'un cinquième signal de retour et le passage à l'étape (v)(b),
(v)(g) lorsque le taux de compression mesuré est inférieur au taux de compression minimal requis, lorsque le compteur de RCP est égal à zéro, le passage à l'étape (vi),
(v)(h) lorsque le taux de compression mesuré est supérieur au taux de compression maximal requis, lorsque le compteur de RCP n'est pas égal à zéro, la diminution du compteur de RCP de 1, la production d'un sixième signal de rétroaction et le passage à l'étape (v)(b),
(v)(i) lorsque le taux de compression mesuré est supérieur au taux de compression maximal requis, lorsque le compteur de RCP est égal à zéro, le passage à l'étape (vi).

11. Défibrillateur (20) selon l'une quelconque des revendications précédentes, ledit système d'évaluation de RCP (26) étant conçu pour effectuer une étape (iii)(a) après l'étape (iii) comprenant la comparaison de la première mesure de performance de RCP à une mesure de performance de RCP minimale, lorsque la première mesure de performance de RCP est supérieure à la mesure de performance de RCP minimale, le passage à l'étape (iv), lorsque la première mesure de performance de RCP est inférieure à la mesure de performance de RCP minimale, la production du troisième signal d'invite et le retour à l'étape (ii).

12. Défibrillateur (20) selon la revendication 11, ledit système d'évaluation de RCP (26) étant conçu pour effectuer une étape (iii)(b) après l'étape (iii)(a) comprenant la comparaison de la première mesure de performance de RCP à une mesure de performance de RCP maximale, lorsque la première mesure de performance de RCP est inférieure à la mesure de performance de RCP maximale, le passage à l'étape (iv), lorsque la première mesure de performance de RCP est supérieure à la mesure de performance de RCP maximale, la production d'un septième signal d'invite et le retour à l'étape (ii).

13. Défibrillateur (20) selon l'une quelconque des revendications précédentes, ledit système d'évaluation de RCP (26) étant conçu pour effectuer une étape (vi)(a) après l'étape (vi) comprenant la comparaison de la mesure supplémentaire de performance de RCP à une mesure de performance de RCP minimale, lorsque la mesure supplémentaire de performance de RCP est supérieure à la mesure de performance de RCP minimale, le passage à l'étape (vii), lorsque la mesure supplémentaire de performance de RCP est inférieure à la mesure de performance de RCP minimale, la production du troisième signal d'invite et le retour à l'étape (v).

14. Défibrillateur (20) selon la revendication 13, ledit système d'évaluation de RCP (26) étant conçu pour effectuer une étape (vi)(b) après l'étape (vi)(a) comprenant la comparaison de la mesure supplémentaire de performance de RCP à une mesure de performance de RCP maximale, lorsque la mesure supplémentaire de performance de RCP est inférieure à la mesure de performance de RCP maximale, le passage à l'étape (vii), lorsque la mesure supplémentaire de performance de RCP est supérieure à la mesure de performance de RCP maximale, la production du septième signal d'invite et le retour à l'étape (v).

15. Défibrillateur (20) selon l'une quelconque des revendications précédentes, ledit système de mesure de RCP (24) mesurant les signaux de compression pendant la première pluralité de compressions thoraciques et pendant la pluralité supplémentaire de compressions thoraciques comprenant des signaux physiologiques sensibles à la compression du sujet pendant les compressions thoraciques.
